(19) Europäisches Patentamt
European Patent Office
Office européen des brevets

(11) EP 1 556 322 B1

(12) FASCICULE DE BREVET EUROPEEN

(45) Date de publication et mention de la délivrance du brevet:
**24.02.2016 Bulletin 2016/08**

(21) Numéro de dépôt: **03778481.6**

(22) Date de dépôt: **28.10.2003**

(51) Int Cl.:
***C07C 46/10** (2006.01)* ***C07C 50/04** (2006.01)*
***G03C 7/392** (2006.01)*

(86) Numéro de dépôt international:
**PCT/FR2003/003206**

(87) Numéro de publication internationale:
**WO 2004/039758 (13.05.2004 Gazette 2004/20)**

# (54) PERLES D'UN COMPOSE PHENOLIQUE ET LEUR PROCEDE D'OBTENTION

PERLEN EINER PHENOLISCHEN VERBINDUNGEN UND VERFAHREN ZU DEREN HERSTELLUNG

BEADS OF A PHENOLIC COMPOUND AND METHOD OF OBTAINING SAME

(84) Etats contractants désignés:
**AT BE BG CH CY CZ DE DK EE ES FI FR GB GR HU IE IT LI LU MC NL PT RO SE SI SK TR**

(30) Priorité: **28.10.2002 FR 0213453**

(43) Date de publication de la demande:
**27.07.2005 Bulletin 2005/30**

(73) Titulaire: **RHODIA CHIMIE**
**75009 Paris (FR)**

(72) Inventeur: **LE THIESSE, Jean-Claude**
**F-42100 ST. ETIENNE (FR)**

(74) Mandataire: **Dutruc-Rosset, Marie-Claude**
**Rhodia Services**
**Direction Propriété Industrielle**
**40, rue de la Haie-Coq**
**93306 Aubervilliers Cedex (FR)**

(56) Documents cités:
**EP-A- 0 277 508** **EP-A- 0 740 954**
**US-A- 4 081 485**

- **PATENT ABSTRACTS OF JAPAN vol. 2000, no. 13, 5 février 2001 (2001-02-05) & JP 2000 302716 A (MITSUI CHEMICALS INC), 31 octobre 2000 (2000-10-31)**

Remarques:
Le dossier contient des informations techniques présentées postérieurement au dépôt de la demande et ne figurant pas dans le présent fascicule.

EP 1 556 322 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

## Description

La présente invention a pour objet une nouvelle présentation d'un composé phénolique sous forme solide. L'invention vise plus particulièrement les perles d'hydroquinone. L'invention se rapporte aussi à la préparation desdites perles.

L'hydroquinone est un produit largement utilisé dans de nombreux domaines d'application en tant qu'inhibiteur de polymérisation ou anti-oxydant dans les élastomères. Un autre domaine d'application est la photographie. Il s'ensuit que c'est un produit de grande consommation.

L'hydroquinone est actuellement disponible sur le marché sous la forme d'une poudre cristallisée sous forme d'aiguilles. Les inconvénients qui en résultent sont la présence de fines qui entraînent des problèmes de poussiérage lors du stockage et de la manipulation de ladite poudre.

Or les poussières d'hydroquinone ne sont pas sans danger par rapport à l'environnement en raison de risques d'explosion et eu égard à l'Homme car cette substance est irritante pour les yeux, les voies respiratoires et peut également provoquer des irritations de la peau lorsqu'elle est mise à son contact.

On a proposé selon JP-A-2002-302716, une technique de granulation de l'hydroquinone qui consiste à faire passer la poudre entre deux rouleaux permettant d'obtenir des tablettes puis de concasser ces tablettes de façon à obtenir des granulés.

L'inconvénient de ce procédé est que des poussières peuvent subsister dans le produit granulé, soit en raison du passage en franchise de cristaux au niveau des rouleaux de la compacteuse, soit par l'attrition des tablettes dans le concasseur. De plus, les granulés sont compacts et leur vitesse de dissolution est très faible par rapport à la poudre initiale.

L'objectif de la présente invention est de fournir une nouvelle présentation d'un composé phénolique notamment de l'hydroquinone permettant de pallier les inconvénients précités.

Plus précisément, la présente invention a pour objet des perles d'un composé phénolique et plus particulièrement des perles d'hydroquinone.

Lesdites perles d'un composé phénolique selon l'invention présentent une solubilité à 90°C d'au moins 500 g/l, sa solubilité étant au moins double entre une température dite chaude choisie entre 80°C et 98°C et une température dite froide choisie entre -30°C et 30°C ; lesdites perles se présentant sous forme de particules solides à forte sphéricité qui ont une taille exprimée par le diamètre médian ($d_{50}$) variant de 300 $\mu$m à 2000 $\mu$m et une porosité interne déterminée au porosimètre à mercure variant entre 0,5 et 0,75 $cm^3$/g, et présentant une résistance à l'attrition variant entre 90 et 100 %.

Lesdites perles ont pour caractéristique d'être résistantes à l'attrition tout en étant poreuses ce qui leur confèrent des propriétés de solubilité.

Dans l'exposé de la présente invention, on entend par "perles", des particules solides à forte sphéricité.

L'invention vise également le procédé de préparation desdites perles dont la caractéristique est de préparer à chaud, une solution aqueuse concentrée d'un composé phénolique, la concentration étant, à la température considérée, au moins égale à 500 g/l, puis de fragmenter la solution en gouttelettes et de refroidir les gouttelettes obtenues dans un courant gazeux de telle sorte qu'elles se solidifient en perles qui sont ensuite récupérées puis séchées.

Par "solution concentrée", on entend une solution ayant une concentration proche de la saturation, de préférence de 80 à 95 % en poids de la limite de solubilité, à la température du dispositif de fractionnement.

Une variante préférée du procédé de l'invention consiste à préparer à chaud une solution concentrée d'un composé phénolique, puis à faire passer la solution dans une buse de façon à former des gouttelettes, à solidifier ces dernières en les laissant tomber dans une tour à contre-courant d'un gaz froid, puis à récupérer les perles obtenues et à les sécher.

Le procédé de l'invention est parfaitement bien adapté à la préparation de perles d'hydroquinone mais il convient également pour tout composé phénolique présentant les caractéristiques suivantes :

- une solubilité à chaud importante: pour une température de référence de 90°C, une solubilité dans l'eau d'au moins 500 g/l, de préférence au moins 1000 g/l ; la borne supérieure ne présentant pas de caractère critique mais elle est généralement inférieure à 15000 g/l,
- une différence de solubilité importante à chaud et à froid. c'est-à-dire entre la température au niveau du dispositif de fragmentation et la température du courant gazeux de refroidissement : la solubilité étant au moins double entre ces deux températures de fonctionnement, de préférence au moins 3 à 5 fois.

Comme exemples de composés phénoliques auxquels peut s'appliquer le procédé de l'invention, on peut mentionner ceux répondant à la formule (I) suivante :

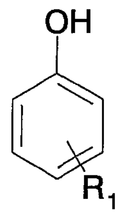

(I)

dans ladite formule (I), $R_1$ représente un groupe hydroxyle, un groupe amino, un groupe alkyle ayant de 1 à 4 atomes de carbone, un groupe alkoxy ayant de 1 à 4 atomes de carbone.

Cette liste de substituants $R_1$ est donné à titre illustratif et sans caractère limitatif dans la mesure où le composé mis en oeuvre présente les caractéristiques physico-chimiques définies ci-dessus.

Comme exemples spécifiques de composés de formule (I), on peut citer tout particulièrement l'hydroquinone, la pyrocatéchine, la résorcine, le m-aminophénol.

L'invention s'applique préférentiellement à l'hydroquinone.

Le procédé de l'invention possède des caractéristiques qui lui sont propres et qui permettent ainsi d'obtenir le composé phénolique sous forme de perles.

Les perles obtenues selon l'invention présentent des caractéristiques physico-chimiques précisées ci-après.

Les définitions et les méthodes de détermination des caractéristiques données ci-après, sont précisées dans les exemples.

Les perles du composé phénolique sont sous la forme de billes de couleur blanche. Ces particules, essentiellement sous forme sphérique, ont un diamètre qui peut être choisi, grâce au procédé de l'invention, dans une large gamme. Ainsi, la taille des particules peut s'échelonner entre 100 μm et 3000 μm mais se situe, de préférence, entre 500 μm et 1500 μm. Précisons que la détermination des tailles se fait par passage sur des tamis métalliques.

La taille des particules exprimée par le diamètre médian ($d_{50}$) est compris entre 300 μm et 2 000 μm, de préférence entre 500 μm et 1500 μm. On définit le diamètre médian comme étant tel que 50 % en poids des particules ont un diamètre supérieur ou inférieur au diamètre médian.

La figure 1 représente une photographie prise au microscope optique qui illustre la morphologie de type perle de l'hydroquinone obtenue selon l'invention. On observe une répartition granulométrique uniforme sur le produit obtenu.

Les perles d'hydroquinone ont une densité qui peut être plus ou moins élevée. La densité apparente (non tassée) des perles est de préférence, d'au moins 0,3 et se situe encore plus préférentiellement entre 0,4 et 0,5. Il y a lieu de noter que les perles de l'invention présentent une densité moins élevée que celle de la poudre cristallisée. Toutefois, elles sont beaucoup moins compressibles car leur taux de compressibilité qui varie entre 5 et 10 % est de 3 à 4 fois inférieur à celui de la poudre cristallisée.

Conformément au procédé de l'invention, on obtient des perles d'un composé phénolique de l'invention qui sont exemptes de poussières et qui ont une forme physique qui leur confère une bonne résistance à l'attrition lors des opérations de transport et de stockage.

La résistance à l'attrition est déterminée par un test réalisé à l'aide d'un tamiseur à courant d'air (type Alpine 200LS-N) équipé d'un tamis ayant une maille de 100 μm. La résistance à l'attrition est exprimée par le rapport entre la masse de perles restant sur le tamis et la masse initiale de perles.

Les perles obtenues présentent une résistance à l'attrition variant entre 90 et 100 %, de préférence supérieure à 95 % et encore plus préférentiellement supérieure à 98 %.

L'invention réside donc en des perles d'un composé phénolique, de préférence d'hydroquinone qui, bien qu'ayant une forme physique leur permettant de résister à l'attrition, conservent une porosité interne importante et, de ce fait, une vitesse de dissolution rapide, lors de leur utilisation.

Il est à noter que les perles présentent une porosité interne déterminée au porosimètre à mercure variant entre 0,5 et 0,75 $cm^3$/g.

Ainsi, on mentionne à titre illustratif, que la vitesse de solubilisation à 20°C, pour une concentration de 5 % en poids d'hydroquinone dans le méthacrylate de méthyle est presque divisée par deux dans le cas des perles par rapport à une poudre. Que l'hydroquinone soit sous forme de poudre ou sous forme de perles selon l'invention, sa vitesse de solubilisation à raison de 2 % dans l'acide acrylique est identique.

La structure originale des produits de l'invention est obtenue grâce à un procédé de fabrication parfaitement adapté.

Le procédé de l'invention de préparation de perles d'un composé phénolique consiste à préparer à chaud, une solution aqueuse concentrée d'un composé phénolique, puis de fragmenter la solution en gouttelettes et refroidir les gouttelettes obtenues dans un courant gazeux de telle sorte qu'elles se solidifient en perles qui sont ensuite récupérées et séchées.

Le procédé de l'invention convient tout à fait bien à la préparation de perles d'hydroquinone. Ledit procédé relève de la technique de prilling mais contrairement à ce qui est habituellement mis en oeuvre, il ne consiste pas à faire fondre l'hydroquinone et à la fragmenter ensuite par passage dans une buse.

En effet, la difficulté à laquelle était confronté l'Homme du Métier était que l'hydroquinone fond à haute température de 172°C et par ailleurs, l'hydroquinone a une tension de vapeur très élevée (supérieure à 25 mbar à cette température) ce qui entraîne à la sortie de buse, une vaporisation phénoménale entraînant des problèmes de poussières et d'assainissement, rédhibitoires d'un point de vue industrielle.

La demanderesse a trouvé qu'il était possible de préparer des perles d'un composé phénolique selon la technique de prilling en partant d'une solution aqueuse d'un composé phénolique.

Conformément au procédé de l'invention, on parvient à obtenir des perles d'un composé phénolique du fait que la solubilité dudit composé, notamment de l'hydroquinone décroît d'une manière importante dès que l'on abaisse la température.

Plus précisément, dans la zone de température définie, le composé phénolique est soluble puis recristallise dès que l'on baisse la température de sa solution aqueuse.

Il importe dans une première étape de préparer une solution aqueuse d'un composé phénolique à une concentration telle qu'elle soit la plus proche de la saturation, à la température considérée.

La température de la solution est choisie suffisamment élevée afin d'obtenir une solubilité au moins égale à 500 g/l, et de préférence 1000 g/l.

Elle est avantageusement choisie entre 80°C et 98°C, de préférence, entre 85°C et 95°C.

Dans cette gamme de températures, on précise à titre indicatif, que la solubilité de l'hydroquinone dans l'eau varie entre 0,9 et 1,7 kg par kg d'eau.

Dans une étape suivante, on transforme la solution d'un composé phénolique, en gouttelettes. Cette opération peut être réalisée au moyen de tout dispositif de fragmentation, par exemple, une turbine, une buse de pulvérisation, une buse plate à orifice(s) circulaire(s).

Un mode de réalisation préférentielle de l'invention consiste à former les gouttelettes par passage de la solution au travers d'un orifice et tout particulièrement par passage au travers d'une buse.

L'opération suivante est d'assurer la solidification des gouttelettes en perles, par contact avec un gaz froid dont la température est choisie entre -30°C et 30°C, de préférence, entre -10°C et 10°C.

Le gaz froid est un gaz quelconque dans la mesure où il est inerte vis-à-vis du composé phénolique, de préférence l'hydroquinone. On choisit de préférence, l'azote ou l'air appauvri en oxygène (par exemple à 10 %).

D'une manière préférentielle, on envoie le courant gazeux froid, à contre-courant du flux de matière.

Le temps de séjour qui est la durée entre la formation de la gouttelette à la sortie de la buse et son arrivée dans le système de récupération se situe avantageusement, entre 1 et 10 secondes et plus préférentiellement entre 3 et 5 secondes.

Une façon d'obtenir le temps de séjour désiré, est de laisser tomber les gouttelettes dans une tour à contre-courant d'un gaz froid tel que précité.

En fin de réaction, on récupère les perles par tout moyen connu, par exemple, par gravité dans un bac de récupération ou selon la technique de lit fluide.

Les perles obtenues se présentent sous la forme solide manipulable mais elles comprennent également de l'eau.

Généralement, les perles de composé phénolique comprennent :

- de 10 à 50 % en poids d'eau,
- de 50 à 90 % en poids de composé phénolique.

Dans le cas de l'hydroquinone, les perles comprennent préférentiellement :

- de 25 à 50 % en poids d'eau,
- de 50 à 75 % en poids d'hydroquinone.

Conformément au procédé de l'invention, on peut procéder dans une étape suivante au séchage des perles obtenues suite à l'opération de prilling.

A cet effet, on soumet les perles à un courant gazeux, de préférence un courant d'air dont la température est comprise entre 20°C (température ambiante) et 90°C, de préférence entre 60°C et 90°C.

Le séchage est avantageusement conduit selon la technique du lit fluide, la température étant progressivement remontée dans la zone de température précitée.

En fin d'opération, on obtient des perles ayant une teneur en eau généralement inférieure à 1 % en poids, comprise entre 0,1 et 1 %, de préférence inférieure à 0,6 %.

En ce qui concerne l'appareillage utilisé pour mettre en oeuvre le procédé de l'invention, il est composé de

deux ensembles : un premier ensemble de mise en forme des perles et un deuxième ensemble de récupération et de séchage des perles.

Le premier ensemble comprend un bac de stockage du composé phénolique équipé de moyens permettant de chauffer la solution du composé phénolique notamment une double enveloppe dans laquelle circule un liquide à la température souhaitée, par exemple de l'eau et une enceinte qui est généralement, une tour comprenant dans sa partie supérieure, un dispositif de fragmentation en gouttelettes, de préférence une buse et équipée dans sa partie inférieure d'une ou plusieurs arrivées d'un courant gazeux froid transformant ainsi le bas de la tour en une tour de refroidissement.

La hauteur de la tour peut varier très largement par exemple, entre 3 et 40 mètres, selon la taille de l'installation. Il est à noter que la borne limite supérieure ne présente aucun caractère critique.

Le composé phénolique et l'eau sont introduites dans un réacteur équipé d'un système permettant de réguler la température, par exemple, une double enveloppe, afin de maintenir ledit composé en solution aqueuse.

La buse utilisée peut être une buse à un seul trou ou une buse multi-trous avec un nombre de trous qui peut varier entre 1 et 3000 trous, et de préférence, entre 1 et 100 trous.

On peut utiliser un système comprenant plusieurs buses, par exemple, 2 buses de préférence amovibles, en parallèle.

Le diamètre des perforations de la buse est fonction de la taille des perles désirées. Il peut être de 50 à 2000 $\mu$m mais il est choisi, de préférence, entre 200 $\mu$m et 600 $\mu$m.

La taille de la perforation est toujours inférieure à la taille de la perle obtenue. Ainsi, on utilise une buse présentant des perforations d'environ 300 $\mu$m pour obtenir des perles présentant un diamètre médian de 500 $\mu$m.

La buse utilisée peut être une buse statique mais il est possible de faire appel à une buse soumise à un système de vibration électrique de haute fréquence, par exemple, de 100 à 10000 hertz. Ce dispositif permet d'obtenir des gouttelettes de taille parfaitement calibrée.

La solution arrive dans la buse de préférence, par une surpression qui est assurée par un courant gazeux, de préférence, un courant d'azote. La surpression par rapport à la pression atmosphérique est de 5 à 500 %.

La buse est maintenue à une température supérieure de 2 à 10°C par rapport à la température de début de cristallisation de la solution aqueuse de composé phénolique.

Au niveau de la buse, il est possible mais non indispensable d'établir un courant gazeux, de préférence un co-courant d'azote avec le jet sortant de la buse. Ce courant gazeux a, de préférence, une température comprise entre la température ambiante et 80°C. La présence de ce co-courant gazeux permet d'obtenir une meilleure régularité de la dimension des perles et évite la coalescence des gouttes.

Dans la partie supérieure de la tour, il peut y avoir sur la paroi interne de la tour, présence de chicanes et de grilles permettant une distribution homogène du flux gazeux.

Dans le bas de la tour, on introduit un courant de gaz froid, de préférence, d'azote ou d'air appauvri en oxygène. Ce courant gazeux froid assure la solidification des gouttelettes en perles. Il a de préférence une température comprise entre -30°C et 30°C, de préférence, comprise entre -10°C et 10°C.

Le courant gazeux froid sort, de préférence, de la tour, en dessous de la buse, à une distance représentant environ un dixième de la hauteur totale de la zone de refroidissement.

Le système de récupération des perles, en bas de la tour, ne présente pas de caractéristique critique. Il peut s'agir d'un bac de récupération ou bien d'un dispositif permettant d'assurer la fluidisation du lit de particules. Il est constitué par un bac, de préférence cylindrique, comportant dans sa partie inférieure, une grille au travers de laquelle est envoyé un courant gazeux, de préférence, d'azote ou d'air appauvri en oxygène. Le débit gazeux, qui dépend de la taille des particules, doit être tel qu'il maintient les particules en suspension. On précise, à titre d'exemple, qu'il est de 5 à 30 $m^3$/h pour un diamètre de lit fluide de 80 mm.

Le dispositif de fluidisation dispose d'une sortie permettant l'évacuation des perles vers tout dispositif de séchage convenant pour effectuer cette opération, notamment un lit fluide ou une étuve.

Un mode de réalisation pratique de l'invention est illustré par le dessin annexé sous forme de figure 2.

La figure 2 est une vue latérale schématique d'un appareil adapté à la mise en oeuvre de l'invention.

L'appareil utilisé est constitué de deux parties : la partie supérieure ou tour de prilling (A) et la partie inférieure qui schématise un dispositif de fluidisation (B).

La solution d'un composé phénolique est introduite dans un bac de stockage à double enveloppe réacteur (1) puis est acheminée vers la buse (2). Pour cela, de l'azote (3) est admis en surpression dans le bac (1).

La tour d'une hauteur de 8 mètres, comprend dans sa partie supérieure une buse (2) éventuellement solidaire d'un vibrateur (4) et est équipée dans sa partie inférieure d'une arrivée d'un courant d'air froid appauvri en oxygène (5).

L'air de refroidissement introduit en (5) ressort de la tour au point (6) en dessous de la buse (2).

Dans la partie supérieure de la tour, des chicanes (7) ainsi qu'une grille (8) de forme annulaire assurent une distribution homogène du flux gazeux dans la tour. Selon une variante non mise en oeuvre dans l'exemple de réalisation, il est possible d'envoyer un flux d'azote (9) chaud ayant une température comprise entre 20°C et 80°C, de préférence entre 60°C et 80°C, distribué à co-courant autour de la buse (2).

Dans la partie inférieure de la tour, une grille de forme tronconique (10) permet de collecter les perles solidifiées dans un dispositif de fluidisation comprenant une arrivée d'air froid en (11) et une sortie (12) permettant l'évacuation en continu des perles obtenues vers un dispositif de séchage de type lit fluide non représenté sur la figure.

On donne ci-après, un exemple de réalisation pratique de l'invention, donné à titre illustratif et sans caractère limitatif.

Avant de détailler les exemples, on précise les méthodes utilisées pour la détermination des différentes caractéristiques des produits obtenus.

- le diamètre médian :

Il est déterminé par passage de la poudre sur tamis.

- la densité apparente tassée et non tassée :

On la mesure sur un appareil illustré par la figure 3.

On commence par peser l'éprouvette vide (2).

On introduit dans l'éprouvette (2) la poudre à mesurer à l'aide de l'entonnoir (1), de manière à ce que le haut du lit de poudre vienne au ras du haut de l'éprouvette jaugée à 250 $cm^3$ (niveau A).

On détermine la masse de la poudre par pesée de l'éprouvette pleine.

On assujettit l'éprouvette sur le support (3) par l'intermédiaire de pinces (4).

On met à zéro le compteur (8) qui totalise le nombre de coups imposés au fond de l'éprouvette.

L'éprouvette est soumise à des chocs verticaux appliqués à sa base par l'intermédiaire d'un marteau (5) actionné par un moteur (6) via une came (7). On arrête l'opération lorsque le volume obtenu est constant (niveau B).

On enregistre l'évolution du volume apparent lu sur les graduations de l'éprouvette en fonction du nombre de coups appliqués à l'aide d'un marteau.

On obtient une courbe expérimentale de tassement.

Volume apparent = f (nombre de coups) que l'on transforme en une courbe densité apparente = f (nombre de coups).

On détermine, avant et après tassement, la densité apparente selon la relation :

$$\text{densité apparente} = \frac{\text{masse de la poudre introduite (g)}}{\text{volume apparent (cm}^3\text{)}}$$

On définit le taux de compressibilité selon la relation :

$$\text{taux de compressibilité} = \frac{\text{densité tassée} - \text{densité non tassée}}{\text{densité tassée}}$$

- la résistance à l'attrition :

La résistance à l'attrition est déterminée par un test réalisé à l'aide d'un tamiseur à courant d'air (type Alpine 200LS-N) équipé d'un tamis ayant une maille de 100 $\mu$m.

Cette maille est choisie car il est communément admis par l'Homme du Métier que ce sont les particules ayant un diamètre inférieur à 100 $\mu$m qui sont susceptibles de générer des poussières lors de la manipulation d'une poudre.

Sous l'action du courant d'air traversant le tamis, les perles sont régulièrement projetées contre le couvercle du tamiseur et soumises à des frictions sur la toile métallique du tamis.

Ces mouvements simulent parfaitement les chocs et les contraintes mécaniques que sont susceptibles de subir les perles durant leur transport et leur stockage.

Le test consiste à placer 40 g de perles sur le tamis de 100 $\mu$m puis à faire fonctionner le tamiseur sous une dépression de 3200 Pa pendant 5 min.

Par pesée de la quantité de perles restant sur le tamis à l'issue du test, on en déduit la quantité de particules de taille inférieure à 100 $\mu$m qui ont été générées au cours du test.

La résistance à l'attrition est exprimée par le rapport entre la masse de perles restant sur le tamis et la masse initiale de perles.

- la porosité interne:

La porosité interne des perles est mesurée au porosimètre à mercure selon la norme ASTM Standards on catalysts D 4284-92.

Exemple :

1. On illustre dans cet exemple, la préparation de perles d'hydroquinone.
2. Elles sont préparées dans un appareillage tel que schématisé par la figure

La buse, non soumise a des vibrations, comporte un trou de 0,5 mm de diamètre et un rapport L/D de 3 ; L représentant la longueur de l'orifice et D le diamètre de l'orifice.

On part de 1500 g d'une poudre d'hydroquinone cristallisée et de 1228 g d'eau, de préférence déminéralisée.

On introduit la poudre d'hydroquinone et l'eau dans le réacteur (1).

On dissout l'hydroquinone dans le réacteur (1) par chauffage à l'aide d'eau chaude circulant dans la double enveloppe. La température du produit est de 94°C en (1) et la température en (2), en sortie de buse est de 92,5°C

La surpression de l'azote en (3) est voisine de 0,1 Bar.

Le débit de solution aqueuse à la sortie de la buse en (2) est de 1,8 kg/h.

On introduit en (5) de l'air de refroidissement à une température de 0°C et à un débit de 850 $m^3/h$, soit une vitesse dans la tour de 0,6 m/s.

L'air ressort en (6) à une température de 3,5°C.

Les perles obtenues sont collectées en (10).

La composition des perles en bas de tour est de 68 % en poids d'hydroquinone et de 32 % en poids d'eau.

Les perles collectées en (10) sont récupérées dans un lit fluide (11). La température de l'air de fluidisation en (11) est de 20°C.

Les perles sont évacuées en (12) pour être ensuite séchées en lit fluide avec de l'air ayant une température comprise entre 60 et 90°C.

La composition des perles obtenues après séchage est de 99,64 % en poids d'hydroquinone et de 0,36 % en poids d'eau.

2. Les caractéristiques des perles obtenues sont les suivantes :

- un diamètre médian (d50) de 1350 μm,
- une densité apparente non tassée de 0,436,
- une densité apparente tassée de 0,462,
- une résistance à l'attrition de 99,5%.
- un taux de compressibilité de 5,6 % (à comparer avec celui de l'hydroquinone cristallisée qui est de 20,4 %),
- une porosité interne de 0,54 $cm^3/g$,
- un temps de solubilisation de :

    . 10 min dans l'acide acrylique à 20°C (idem pour l'hydroquinone cristallisée),
    . et de 1 min 45 s dans le méthacrylate de méthyle à 20°C (3 min pour l'hydroquinone cristallisée).

## Revendications

1. Perles d'un composé phénolique présentant une solubilité à 90°C d'au moins 500 g/l, sa solubilité étant au moins double entre une température dite chaude choisie entre 80°C et 98°C et une température dite froide choisie entre -30°C et 30°C ; lesdites perles se présentant sous forme de particules solides à forte sphéricité qui ont une taille exprimée par le diamètre médian ($d_{50}$) variant de 300 μm à 2000 μm et une porosité interne déterminée au porosimètre à mercure variant entre 0,5 et 0,75 $cm^3/g$, et présentant une résistance à l'attrition variant entre 90 et 100 %.

2. Perles selon la revendication 1 **caractérisées par le fait que** le composé phénolique présente une solubilité à 90°C d'au moins 1000 g/l.

3. Perles selon l'une des revendications 1 et 2 **caractérisées par le fait que** le composé phénolique présente une différence de solubilité importante à chaud et à froid : la solubilité étant au moins 3 à 5 fois supérieure entre ladite température chaude et ladite température froide.

4. Perles selon l'une des revendications 1 à 3 **caractérisé par le fait que** le composé phénolique répond à la formule (I) suivante :

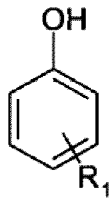
(I)

dans ladite formule (I), $R_1$ représente un groupe hydroxyle, un groupe amino, un groupe alkyle ayant de 1 à 4 atomes de carbone, un groupe alkoxy ayant de 1 à 4 atomes de carbone.

5. Perles selon l'une des revendications 1 à 4 **caractérisé par le fait que** le composé phénolique est choisi parmi l'hydroquinone, la pyrocatéchine, la résorcine, le m-aminophénol, de préférence l'hydroquinone.

6. Perles selon l'une des revendications 1 à 5 **caractérisées par le fait qu'**elles ont une taille exprimée par le diamètre médian ($d_{50}$) variant de 500 $\mu$m à 1500 $\mu$m.

7. Perles selon l'une des revendications 1 à 6 **caractérisées par le fait qu'**elles présentent une résistance à l'attrition supérieure à 95 %, et encore plus préférentiellement supérieure à 98 %.

8. Perles selon la revendication 5 **caractérisées par le fait qu'**elles ont une densité apparente (non tassée) d'au moins 0,3 et plus préférentiellement entre 0,4 et 0,5.

9. Perles selon la revendication 5 **caractérisées par le fait qu'**elles ont un taux de compressibilité de 5 à 10 %.

10. Perles d'hydroquinone selon la revendication 1 **caractérisées par le fait qu'**elles se présentent sous la forme de particules solides à forte sphéricité ayant un diamètre médian ($d_{50}$) variant de 300 $\mu$m à 2000 $\mu$m, une porosité interne déterminée au porosimètre à mercure variant entre 0,5 et 0,75 $cm^3/g$, une résistance à l'attrition variant entre 90 et 100 %.

11. Perles d'hydroquinone selon la revendication 10 **caractérisées par le fait qu'**elles se présentent sous la forme de particules solides à forte sphéricité ayant un diamètre médian ($d_{50}$) variant de entre 500 $\mu$m et 1500 $\mu$m, une porosité interne déterminée au porosimètre à mercure variant entre 0,5 et 0,75 $cm^3/g$, une résistance à l'attrition supérieure à 95 %, de préférence supérieure à 98 %.

12. Procédé de préparation des perles décrites dans l'une des revendications 1 à 11 **caractérisé par le fait qu'**il consiste à préparer à chaud, une solution aqueuse concentrée d'un composé phénolique, la concentration étant, à la température considérée, au moins égale à 500 g/l, puis de fragmenter la solution en gouttelettes et de refroidir les gouttelettes obtenues dans un courant gazeux de telle sorte qu'elles se solidifient en perles qui sont ensuite récupérées puis séchées.

13. Procédé selon la revendication 12 **caractérisé par le fait qu'**il consiste à faire passer la solution d'un composé phénolique dans une buse de façon à former des gouttelettes, à solidifier ces dernières en les laissant tomber dans une tour à contre-courant d'un gaz froid puis à récupérer les perles obtenues.

14. Procédé selon l'une des revendications 12 et 13 **caractérisé par le fait que** l'on prépare une solution aqueuse d'un composé phénolique à une concentration, à la température considérée, au moins égale à 1 000 g/l.

15. Procédé selon l'une des revendications 12 à 14 **caractérisé par le fait que** la température de la solution est choisie suffisamment élevée afin d'obtenir une solubilité désirée et est choisie de préférence entre 80°C et 98°C, et encore plus préférentiellement, entre 85°C et 95°C.

**16.** Procédé selon la revendication 13 **caractérisé par le fait que** la buse utilisée est une buse à un seul trou ou une buse multi-trous avec un nombre de trous variant entre 1 et 3000 trous, de préférence entre 1 et 100 trous.

**17.** Procédé selon la revendication 16 **caractérisé par le fait que** la buse utilisée comporte des perforations dont le diamètre varie entre 50 à 2000 $\mu$m et se situe, de préférence, entre 200 et 600 $\mu$m.

**18.** Procédé selon l'une des revendications 16 et 17 **caractérisé par le fait que** la buse utilisée est une buse statique mais de préférence une buse soumise à un système de vibration électrique de haute fréquence, de préférence, de 100 à 10000 hertz.

**19.** Procédé selon l'une des revendications 12 à 18 **caractérisé par le fait que** les gouttelettes sont mises en contact avec un gaz froid, de préférence d'azote ou d'air appauvri en oxygène dont la température est choisie entre -30°C et 30°C, de préférence, entre -10°C et 10°C.

**20.** Procédé selon l'une des revendications 12 à 19 **caractérisé par le fait que** le temps de séjour de la gouttelette à la sortie de la buse et son arrivée dans le système de récupération se situe de préférence, entre 1 et 10 secondes et plus préférentiellement entre 3 et 5 secondes.

**21.** Procédé selon l'une des revendications 12 à 20 **caractérisé par le fait que** l'on récupère les perles de préférence selon la technique de lit fluide.

**22.** Procédé selon l'une des revendications 12 à 21 **caractérisé par le fait que** la composition des perles de composé phénolique au bas de la tour de prilling est :

- de 10 à 50 %, en poids d'eau,
- de 50 à 90 %, en poids de composé phénolique.

**23.** Procédé selon l'une des revendications 12 à 22 **caractérisé par le fait que** la composition des perles d'hydroquinone au bas de la tour de prilling est :

- de 25 à 50 % en poids d'eau,
- de 50 à 75 % en poids d'hydroquinone.

**24.** Procédé selon l'une des revendications 12 à 23 **caractérisé par le fait que** l'on soumet les perles à un courant gazeux, de préférence un courant d'air dont la température est comprise entre 20°C et 90°C, de préférence entre 60°C et 90°C.

**25.** Procédé selon la revendication 24 **caractérisé par le fait que** le séchage est conduit selon la technique du lit fluide.

**26.** Procédé selon l'une des revendications 24 et 25 **caractérisé par le fait que** la composition des perles de composé phénolique après séchage est :

- de 0,1 à 1 % en poids d'eau,
- de 99 à 99,9 % en poids de composé phénolique.

**27.** Procédé selon la revendication 26 **caractérisé par le fait que** la composition des perles de composé phénolique après séchage est :

- de 0,1 à 0,6 % en poids d'eau,
- de 99,4 à 99,9 % en poids de composé phénolique.

### Patentansprüche

**1.** Perlen einer phenolischen Verbindung, die bei 90 °C eine Löslichkeit von mindestens 500 g/l aufweisen, wobei ihre Löslichkeit zwischen einer so genannten heißen Temperatur, die zwischen 80 °C und 98 °C ausgewählt ist, und einer so genannten kalten Temperatur, die zwischen -30 °C und 30 °C ausgewählt ist, mindestens doppelt so groß ist; wobei die Perlen in Form von festen Partikeln mit großer Sphärizität vorliegen, die eine Größe, ausgedrückt

durch den mittleren Durchmesser ($d_{50}$), aufweisen, der von 300 μm bis 2.000 μm variiert, und eine innere Porosität, bestimmt mittels Quecksilber-Porosimeter, die zwischen 0,5 und 0,75 $cm^3/g$ variiert, und eine Abriebfestigkeit aufweisen, die zwischen 90 und 100 % variiert.

**2.** Perlen nach Anspruch 1, **dadurch gekennzeichnet, dass** die phenolische Verbindung bei 90 °C eine Löslichkeit von mindestens 1.000 g/l aufweist.

**3.** Perlen nach einen der Ansprüche 1 und 2, **dadurch gekennzeichnet, dass** die phenolische Verbindung bei Wärme und bei Kälte eine stark unterschiedliche Löslichkeit aufweist: wobei die Löslichkeit zwischen der warmen Temperatur und der kalten Temperatur mindestens 3 bis 5 Mal größer ist.

**4.** Perlen nach einem der Ansprüche 1 bis 3, **dadurch gekennzeichnet, dass** die phenolische Verbindung der folgenden Formel (I) entspricht:

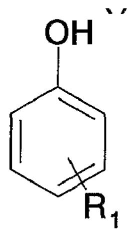

(I)

wobei in dieser Formel (I) $R_1$ für eine Hydroxylgruppe, eine Aminogruppe, eine Alkylgruppe mit 1 bis 4 Kohlenstoffatomen, eine Alkoxygruppe mit 1 bis 4 Kohlenstoffatomen steht.

**5.** Perlen nach einem der Ansprüche 1 bis 4, **dadurch gekennzeichnet, dass** die phenolische Verbindung aus Hydrochinon, Pyrocatechin, Resorcin, m-Aminophenol, bevorzugt Hydrochinon, ausgewählt ist.

**6.** Perlen nach einem der Ansprüche 1 bis 5, **dadurch gekennzeichnet, dass** sie eine Größe, ausgedrückt durch den mittleren Durchmesser ($d_{50}$), aufweisen, die zwischen 500 μm bis 1.500 μm variiert.

**7.** Perlen nach einem der Ansprüche 1 bis 6, **dadurch gekennzeichnet, dass** sie eine Abriebfestigkeit größer 95 %, und noch stärker bevorzugt größer 98 % aufweisen.

**8.** Perlen nach Anspruch 5, **dadurch gekennzeichnet, dass** sie eine Rohdichte (Schüttdichte) von mindestens 0,3 und stärker bevorzugt zwischen 0,4 und 0,5 aufweisen.

**9.** Perlen nach Anspruch 5, **dadurch gekennzeichnet, dass** sie einen Kompressibilitätsgrad von 5 bis 10 % aufweisen.

**10.** Hydrochinonperlen nach Anspruch 1, **dadurch gekennzeichnet, dass** sie in Form von festen Partikeln mit großer Sphärizität mit einem mittleren Durchmesser ($d_{50}$), der zwischen 300 μm bis 2.000 μm variiert, einer inneren Porosität, bestimmt mittels Quecksilberporosimeter, die zwischen 0,5 und 0,75 $cm^3/g$ variiert, einer Abriebfestigkeit, die zwischen 90 und 100 % variiert, vorliegen.

**11.** Hydrochinonperlen nach Anspruch 10, **dadurch gekennzeichnet, dass** sie in Form von festen Partikeln mit großer Sphärizität mit einem mittleren Durchmesser ($d_{50}$), der zwischen 500 μm und 1.500 μm variiert, einer inneren Porosität, bestimmt mittels Quecksilberporosimeter, die zwischen 0,5 und 0,75 $cm^3/g$ variiert, einer Abriebfestigkeit größer 95 %, bevorzugt größer 98 %, vorliegen.

**12.** Verfahren zur Herstellung der in einem der Ansprüche 1 bis 11 beschriebenen Perlen, **dadurch gekennzeichnet, dass** es darin besteht, eine konzentrierte wässrige Lösung einer phenolischen Verbindung heiß herzustellen, wobei die Konzentration bei der betreffenden Temperatur mindestens gleich 500 g/l beträgt, die Lösung dann in Tröpfchen aufzuteilen und die erhaltenen Tröpfchen in einem Gasstrom derart abzukühlen, dass sie sich zu Perlen verfestigen, die anschließend gewonnen, dann getrocknet werden.

**13.** Verfahren nach Anspruch 12, **dadurch gekennzeichnet, dass** es darin besteht, die Lösung einer phenolischen Verbindung durch eine Düse zu leiten, um Tröpfchen zu bilden, letztere zu verfestigen, indem man sie in einen Turm mit Kaltgas-Gegenstrom fallen lässt, die erhaltenen Perlen dann zu gewinnen.

**14.** Verfahren nach einem der Ansprüche 12 und 13, **dadurch gekennzeichnet, dass** eine wässrige Lösung einer Phenolverbindung mit einer Konzentration, bei der betreffenden Temperatur, von mindestens gleich 1.000 g/l hergestellt wird.

**15.** Verfahren nach einem der Ansprüche 12 bis 14, **dadurch gekennzeichnet, dass** die Temperatur der Lösung ausreichend hoch ausgewählt wird, um eine gewünschte Löslichkeit zu erhalten, und bevorzugt zwischen 80 °C und 98 °C, und noch stärker bevorzugt zwischen 85 °C und 95 °C ausgewählt wird.

**16.** Verfahren nach Anspruch 13, **dadurch gekennzeichnet, dass** die verwendete Düse eine Düse mit einem einzigen Loch oder eine Mehrlochdüse mit einer Anzahl von Löchern ist, die zwischen 1 und 3.000 Löchern, bevorzugt zwischen 1 und 100 Löchern variiert.

**17.** Verfahren nach Anspruch 16, **dadurch gekennzeichnet, dass** die verwendete Düse Perforationen umfasst, deren Durchmesser zwischen 50 bis 2.000 $\mu$m variiert und bevorzugt zwischen 200 und 600 $\mu$m liegt.

**18.** Verfahren nach einem der Ansprüche 16 und 17, **dadurch gekennzeichnet, dass** die verwendete Düse eine statische Düse ist, bei der es sich aber bevorzugt um eine Düse handelt, die mit einem elektrischen Hochfrequenzvibrationssystem, bevorzugt 100 bis 10.000 Hertz, beaufschlagt ist.

**19.** Verfahren nach einem der Ansprüche 12 bis 18, **dadurch gekennzeichnet, dass** die Tröpfchen mit einem kalten Gas, bevorzugt Stickstoff oder sauerstoffabgereicherter Luft, dessen/deren Temperatur zwischen -30 °C und 30 °C, bevorzugt zwischen -10 °C und 10 °C ausgewählt ist, in Kontakt gebracht werden.

**20.** Verfahren nach einem der Ansprüche 12 bis 19, **dadurch gekennzeichnet, dass** die Verweildauer des Tröpfchens am Ausgang der Düse und ihre Ankunft in dem Gewinnungssystem bevorzugt zwischen 1 und 10 Sekunden und noch stärker bevorzugt zwischen 3 und 5 Sekunden liegt.

**21.** Verfahren nach einem der Ansprüche 12 bis 20, **dadurch gekennzeichnet, dass** die Perlen bevorzugt gemäß der Fließbetttechnik gewonnen werden.

**22.** Verfahren nach einem der Ansprüche 12 bis 21, **dadurch gekennzeichnet, dass** die Zusammensetzung der Perlen aus der phenolischen Verbindung am Boden des Sprühkondensationsturms folgendermaßen ist:

- 10 bis 50 Gew.-% Wasser,
- 50 bis 90 Gew.-% der phenolischen Verbindung.

**23.** Verfahren nach einem der Ansprüche 12 bis 22, **dadurch gekennzeichnet, dass** die Zusammensetzung der Hydrochinonperlen am Boden des Sprühkondensationsturms folgendermaßen ist:

- 25 bis 50 Gew.-% Wasser,
- 50 bis 75 Gew.-% Hydrochinon.

**24.** Verfahren nach einem der Ansprüche 12 bis 23, **dadurch gekennzeichnet, dass** die Perlen mit einem Gasstrom, bevorzugt einem Luftstrom, beaufschlagt werden, dessen Temperatur im Bereich zwischen 20 °C und 90 °C, bevorzugt zwischen 60 °C und 90 °C liegt.

**25.** Verfahren nach Anspruch 24, **dadurch gekennzeichnet, dass** das Trocknen gemäß der Fließbetttechnik durchgeführt wird.

**26.** Verfahren nach einem der Ansprüche 24 und 25, **dadurch gekennzeichnet, dass** die Zusammensetzung der Perlen aus der phenolischen Verbindung nach dem Trocknen folgendermaßen ist:

- 0,1 bis 1 Gew.-% Wasser,
- 99 bis 99,9 Gew.-% der phenolischen Verbindung.

**27.** Verfahren nach Anspruch 26, **dadurch gekennzeichnet, dass** die Zusammensetzung der Perlen aus der phenolischen Verbindung nach dem Trocknen folgendermaßen ist:

- 0,1 bis 0,6 Gew.-% Wasser,
- 99,4 bis 99,9 Gew.-% der phenolischen Verbindung.

**Claims**

**1.** Beads of a phenolic compound having a solubility at 90°C of at least 500 g/l, the solubility thereof being at least double between a "hot" temperature chosen between 80°C and 98°C and a "cold" temperature chosen between -30°C and 30°C; said beads being in the form of highly spherical solid particles having a size, expressed by the median diameter ($d_{50}$), varying from 300 μm to 2000 μm and an internal porosity, determined on a mercury porosimeter, varying between 0.5 and 0.75 $cm^3/g$ and having a resistance to attrition varying between 90 and 100%.

**2.** Beads according to Claim 1, **characterized in that** the phenolic compound has a solubility at 90°C of at least 1000 g/l.

**3.** Beads according to either of Claims 1 and 2, **characterized in that** the phenolic compound has a large difference in hot and cold solubility, with the solubility being at least 3 to 5 times higher between said hot temperature and said cold temperature.

**4.** Beads according to one of Claims 1 to 3, **characterized in that** the phenolic compound corresponds to the following formula (I):

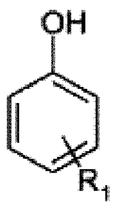

(I)

in said formula (I), $R_1$ represents a hydroxyl group, an amino group, an alkyl group having from 1 to 4 carbon atoms or an alkoxy group having from 1 to 4 carbon atoms.

**5.** Beads according to one of Claims 1 to 4, **characterized in that** the phenolic compound is chosen from hydroquinone, pyrocatechol, resorcinol, m-aminophenol, preferably hydroquinone.

**6.** Beads according to one of Claims 1 to 5, **characterized in that** they have a size, expressed by the median diameter ($d_{50}$), varying from 500 μm to 1500 μm.

**7.** Beads according to one of Claims 1 to 6, **characterized in that** they have a resistance to attrition of greater than 95%, and even more preferably of greater than 98%.

**8.** Beads according to Claim 5, **characterized in that** they have a bulk density (loose) of at least 0.3 and more preferably between 0.4 and 0.5.

**9.** Beads according to Claim 5, **characterized in that** they have a degree of compressibility of from 5 to 10%.

**10.** Hydroquinone beads according to Claim 1, **characterized in that** they are in the form of highly spherical solid particles having a median diameter ($d_{50}$) varying from 300 μm to 2000 μm, an internal porosity, determined on a mercury porosimeter, varying between 0.5 and 0.75 $cm^3/g$ and a resistance to attrition varying between 90 and 100%.

**11.** Hydroquinone beads according to Claim 10, **characterized in that** they are in the form of highly spherical solid particles having a median diameter ($d_{50}$) varying between 500 μm and 1500 μm, an internal porosity, determined on a mercury porosimeter, varying between 0.5 and 0.75 $cm^3/g$ and a resistance to attrition of greater than 95%, preferably of greater than 98%.

**12.** Process for preparing the beads described in one of Claims 1 to 11, **characterized in that** the process consists in preparing, under hot conditions, a concentrated aqueous solution of a phenolic compound, the concentration being, at the temperature in question, at least equal to 500 g/l, then in fragmenting the solution into droplets and in cooling the droplets obtained in a stream of gas such that they solidify into beads which are then recovered and then dried.

**13.** Process according to Claim 12, **characterized in that** it consists in conveying the solution of a phenolic compound into a nozzle so as to form droplets, in solidifying the latter by dropping them into a tower in countercurrent to a cold gas and then in recovering the beads obtained.

**14.** Process according to either of Claims 12 and 13, **characterized in that** an aqueous solution of a phenolic compound is prepared at a concentration, at the temperature in question, at least equal to 1000 g/l.

**15.** Process according to one of Claims 12 to 14, **characterized in that** the temperature of the solution is chosen to be sufficiently high so as to obtain the desired solubility and is preferably chosen to be between 80°C and 98°C, and even more preferably between 85°C and 95°C.

**16.** Process according to Claim 13, **characterized in that** the nozzle used is a single-hole or multi-hole nozzle with a number of holes varying between 1 and 3000 holes, preferably between 1 and 100 holes.

**17.** Process according to Claim 16, **characterized in that** the nozzle used comprises perforations, the diameter of which varies between 50 and 2000 μm and is preferably between 200 and 600 μm.

**18.** Process according to either of Claims 16 and 17, **characterized in that** the nozzle used is a static nozzle but is preferably a nozzle subjected to a highfrequency electrical vibration system, preferably from 100 to 10 000 Hz.

**19.** Process according to one of Claims 12 to 18, **characterized in that** the droplets are brought into contact with a cold gas, preferably nitrogen or oxygen-depleted air, the temperature of which is chosen to be between -30°C and 30°C and preferably between -10°C and 10°C.

**20.** Process according to one of Claims 12 to 19, **characterized in that** the time spent by the droplet at the outlet of the nozzle and its arrival in the recovery system is preferably between 1 and 10 seconds and more preferably between 3 and 5 seconds.

**21.** Process according to one of Claims 12 to 20, **characterized in that** the beads are preferably recovered according to the fluid bed technique.

**22.** Process according to one of Claims 12 to 21, **characterized in that** the composition of the beads of phenolic compound at the bottom of the prilling tower is:

- from 10 to 50% by weight of water,
- from 50 to 90% by weight of phenolic compound.

**23.** Process according to one of Claims 12 to 22, **characterized in that** the composition of the hydroquinone beads at the bottom of the prilling tower is:

- from 25 to 50% by weight of water,
- from 50 to 75% by weight of hydroquinone.

**24.** Process according to one of Claims 12 to 23, **characterized in that** the beads are subjected to a stream of gas, preferably a stream of air, the temperature of which is between 20°C and 90°C, preferably between 60°C and 90°C.

**25.** Process according to Claim 24, **characterized in that** the drying is carried out according to the fluid bed technique.

**26.** Process according to either of Claims 24 and 25, **characterized in that** the composition of the beads of phenolic compound after drying is:

- from 0.1 to 1% by weight of water,
- from 99 to 99.9% by weight of phenolic compound.

**27.** Process according to Claim 26, **characterized in that** the composition of the beads of phenolic compound after drying is:

- from 0.1 to 0.6% by weight of water,
- from 99.4 to 99.9% by weight of phenolic compound.

Figure 1

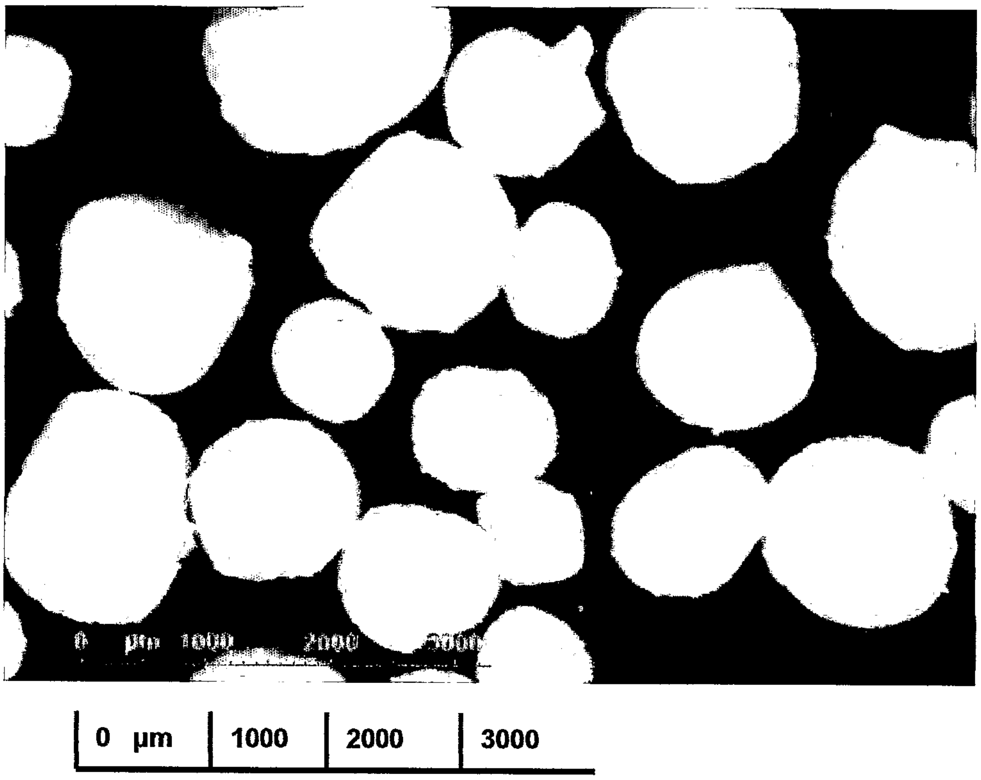

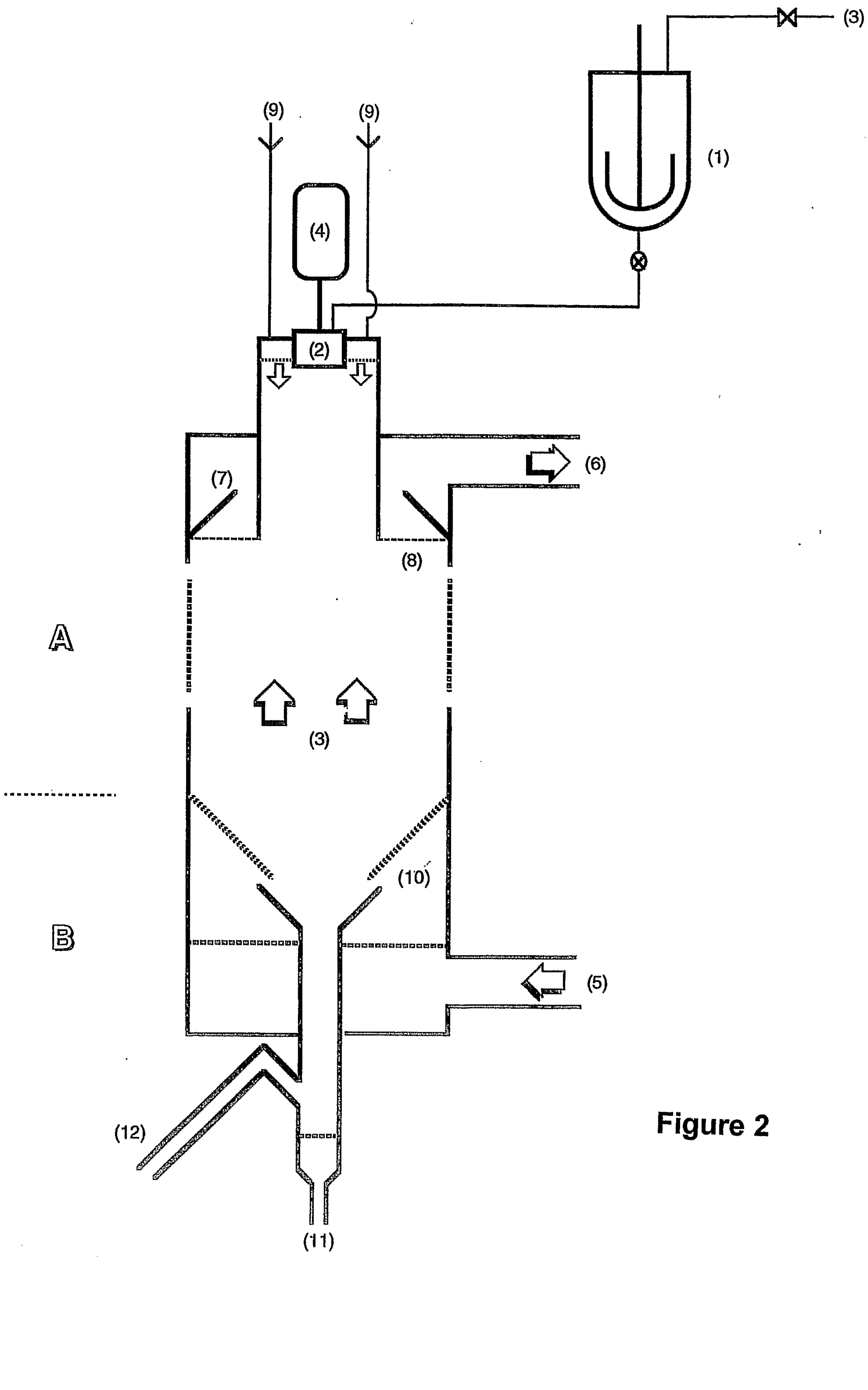


Figure 2

**Figure 3**

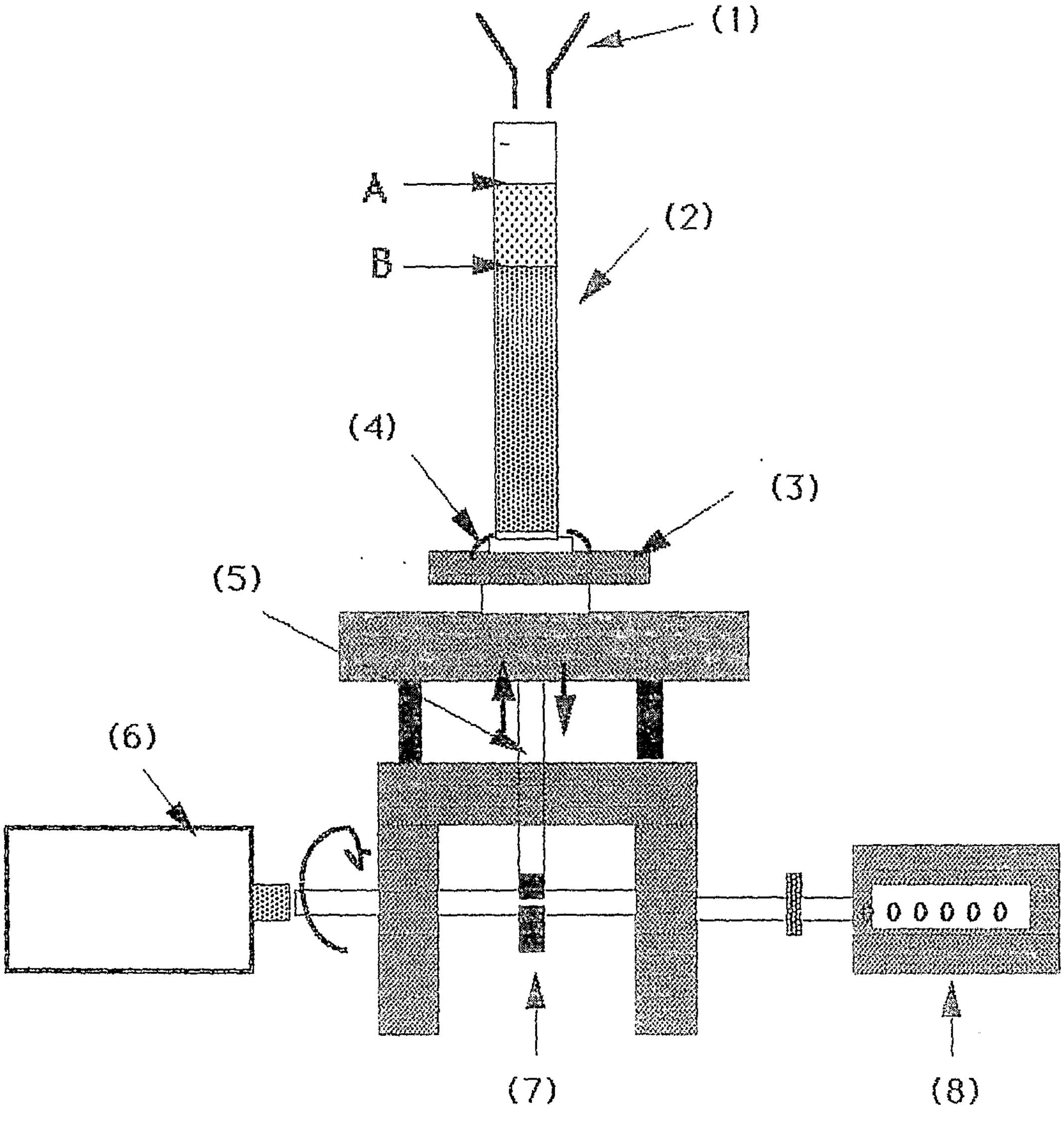

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- JP 2002302716 A